(19) Europäisches Patentamt
European Patent Office
Office européen des brevets

(11) EP 1 552 829 A1

(12) EUROPEAN PATENT APPLICATION
published in accordance with Art. 158(3) EPC

(43) Date of publication:
**13.07.2005 Bulletin 2005/28**

(21) Application number: **03797149.6**

(22) Date of filing: **04.09.2003**

(51) Int Cl.[7]: **A61K 31/335**, C07D 493/22, A61P 37/06

(86) International application number:
**PCT/CN2003/000748**

(87) International publication number:
**WO 2004/026298 (01.04.2004 Gazette 2004/14)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **18.09.2002 CN 02130686**

(71) Applicants:
- **FARREACH LAB.**
  **Chengdu, Sichuan 610041 (CN)**
- **W & K INTERNATIONAL, INC.**
  **Mount Laurel, NJ 08054 (US)**

(72) Inventors:
- **WANG, Dayuan**
  **Mt. Laurel, NJ 08054 (US)**
- **TAN, Hong**
  **Chengdu Sichuan 610041 (CN)**
- **KONG, Yan**
  **Shanghai 200041 (CN)**

(74) Representative: **Ferreccio, Rinaldo**
**c/o Botti & Ferrari S.r.l.**
**Via Locatelli 5**
**20124 Milano (IT)**

# (54) DERIVATIVES OF TRIPTOLIDE HAVING HIGH IMMUNOSUPPRESSIVE EFFECT AND HIGH WATER SOLUBILITY, AND USES THEREOF

(57) The present invention provides water soluble triptolide derivatives of formula I, II, IIIa, and IIIb, which have high immunosuppressive activity and in which $R_1$ and $R_2$ have the same meanings as defined in claims. The present invention also provides chemically synthetic methods of the triptolide derivatives of formula I, II, IIIa, and IIIb, and the use of the triptolide derivatives in the treatment of autoimmune deficiency diseases and inflammatory diseases correlated with immunosuppression.

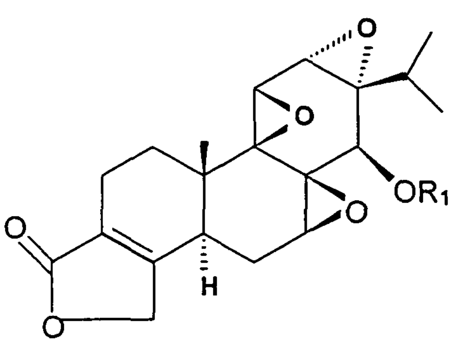

I

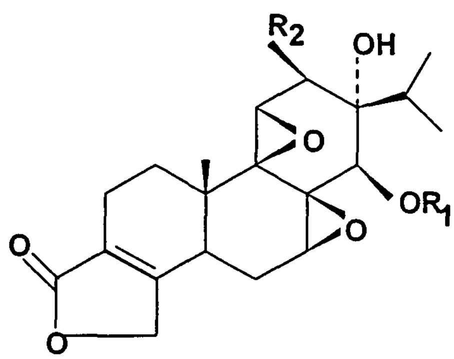

II

EP 1 552 829 A1

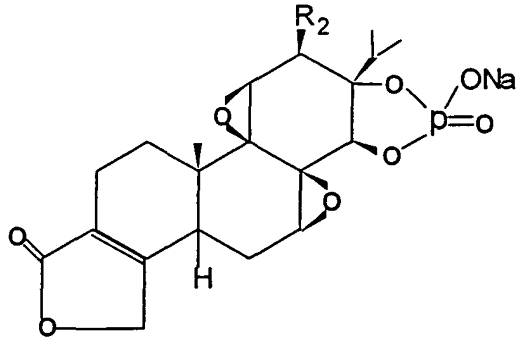

IIIa

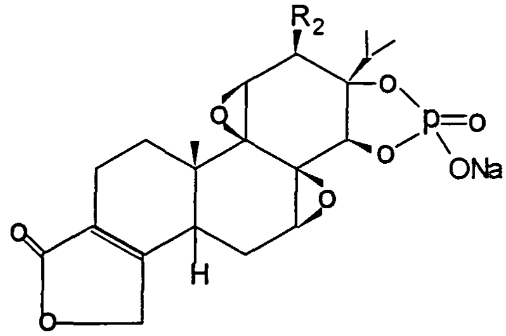

IIIb

**Description**

## Field of the Invention

The present invention relates to pharmaceutical chemistry, and more particularly to water-soluble derivatives of triptolide with high immunosuppressive activity, preparation methods thereof, and their use in the treatment of diseases correlated with immunosuppression.

## Background of the Invention

Immunosuppressive agents are widely used in the treatment of autoimmune deficiency diseases and immuno-related inflammatory diseases such as rheumatoid arthritis, asthma, systemic lupus erythematosus (SLE), psoriasis, multiple sclerosis, atherosclerosis, nephritis, and type I diabetes. Immunosuppressive agent is also the most effective therapeutic mean for the immunological rejection after organ transplant. The most widely used immunosuppressive agents comprise azathyopurine, corticosterroids, methotrexate, cyclophosphamide, 6-mercaptopurine, vincristine, celebrox, cyclosporine A, FK506, etc. But not all of these drugs are completely effective and most of them have severe toxicity.

Immunosuppressive activity is closely related to lymphocytes, especially T and B cells. A number of cytokines are secreted by T and B cells. Each of these cytokines has its specific physiological role and their roles in therapeutic areas vary accordingly. It is insufficient to conclude a compound has high or low immunosuppressive activity just by simply detecting the inhibitory effect of the compound on T and B cells. In contrast, the inhibitory effect on T and B cells is presently used to detect the cytotoxicity of compound. For example, cyclosporine A and methotrexate both as immunosuppressive agents inhibit different cytokines. Cyclosporine A mainly inhibits interleukin II (IL-2) and has little inhibition on IL-1, and its clinical application is mainly to prevent transplant rejection after organ transplantation. In contrast, methotrexate has weak inhibitory effect on IL-II, however strong inhibition on IL-I, and is mainly applied to the treatment of inflammatory diseases correlated with immuno-suppression, such as rheumatoid arthritis. Although more than a dozen of cytokines have been discovered, the physiological functions of most of the cytokines in immune-regulation are not very clear. The immune-regulating and anti-inflammatory functions of the cytokines such as IL-1, IL-2, IL-6 and other inflammatory agents such as TNF, iNOS and Cox-2 have been widely studied, and their therapeutic uses are relatively clearer than those of others. Therefore, the study and evaluation of functions of these cytokines and anti-inflammatory agents before an animal experiment will be very helpful for the design of the objective and experimental procedure of the animal experiment thereafter.

The Chinese medicinal plant *Tripterygium wilfordii* (Leigongteng) has proved to have a very high immunosuppressive activity. The crude extract of *Tripterygium wilfordii* (marketed polyglycoside tablet of *Tripterygium wilfordii*) has been applied in clinic trials to treat autoimmune deficiency diseases such as rheumatoid arthritis, asthma, systemic lupus erythematosus (SLE), psoriasis. Triptolide, a compound extracted from *Tripterygium wilfordii*, has demonstrated its activity of prevention of transplant rejection after organ grafting, which is comparable to cyclosporine A. The significant immunosuppressive activity of *Tripterygium wilfordii* has drawn attention from a number of laboratories all over the world, and these labs have conducted a series of studies:

1. Isolation of efficient immunosuppressive agents from *Tripterygium wilfordii.* More than 100 compounds including triptolide, 16-OH triptolide, triptophenolide, tripdiolide, tripchorolide have hitherto been identified and isolated. Among them, lots of studies have been conducted on the immunoregulation and anti-tumor activities of triptolide. Triptolide has been widely investigated for its highest immunosuppressive activity, relatively high content in plants and more sites for further structure modification.

2. Chemical structure modification of triptolide. The purpose of the structure modification is to reduce its cytotoxicity. The following are the relevant patent applications:

   US Patent Nos. 6,150,539; 6,004,999; 5,972,998; 5,962,516; and 5,666,335;
   PCT application: WO 00/12483;
   Chinese Patent: CN 1027371C, ZL 89106941.0

3. Chemical total synthesis of triptolide:

   Yang D et.al, *J. Org. Chem.* 2000, Apr 7, 65(7):2208-17.

4. Triptolide production via plant cell culture

US Patent No. 4,328,309

Despite of quite high immunosuppressive activity, triptolide has been limited in its clinic application for the following drawbacks: (1) High toxicity with a $LD_{50}$ of 0.85 mg/kg body weight, and genital toxicity. (2) Low water solubility and unsuitability for i.v. (intravenous) injections. (3) Low cell membrane permeability (our unpublished experimental data) and unsatisfactory oral administration.

As for these problems, several laboratories have conducted a serial of work on the chemical structure modification of triptolide. For example, US Patent No. 5,962,516 discloses 14-succinyl triptolide, which has an improved water solubility. Both 8-hydroxy triptolid disclosed in US Patent No. 6,150,539 and 12-thiocyano-triptolide disclosed in PCT application WO 00/63212 have succeeded in possessing less toxicity than the lead compound, triptolide, while keeping activity of original compound. The toxicity of 12-thiocyano-triptolide with a $LD_{50}$ of 74 mg/kg body weight is decreased by about 87 times compared with the lead compound, triptolide, which has a $LD_{50}$ of 0.85 mg/kg body weight. But these analogs derived from triptolide still have low water solubility and low cell permeability.

PCT application WO 02/28862 provides some information about water-soluble derivatives of triptolide. US Patent No. 6,150,539 provides several water-soluble derivatives of triptolide, but no experimental data of immunosuppressive activity of these derivatives has been showed therein. At present, 14-succinyl triptolide disclosed in US Patent No. 5,962,516 represents the best derivative of triptolide in terms of immunosuppressive activity and water solubility. This compound has proved its use in anti-tumor and organ transplantation in US Patent No. 6,329,148 and *Transplantation*, 70(10): 1442-7, Nov 27, 2000, respectively.

## Description of the Invention

In one aspect, the invention provides three types of novel derivatives of triptolide with high water-solubility and immunosuppressive activity.

The first type of novel derivatives of triptolide is as follow:

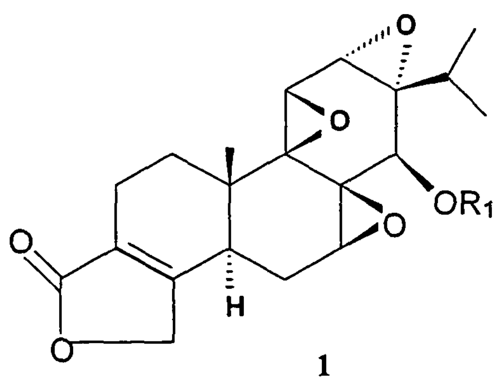

**1**

wherein $R_1$ is H, phosphate

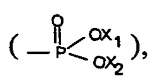

or phosphite

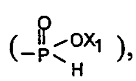

preferably phosphate

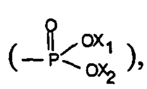

wherein $X_1$ and $X_2$ are Na.

The second type of novel derivatives of triptolide is as follow:

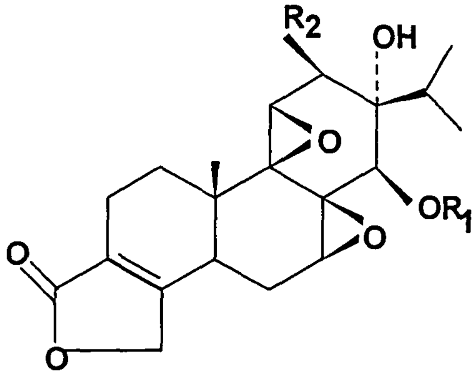

II

wherein $R_1$ is H, alkyl having 1-4 carbon atom(s), -Ac, $-C(=O)(CH_2)_nCO_2$ (n is an integer of 1-4), phosphate

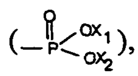

or phosphite

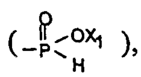

wherein $X_1$ and $X_2$ are Na, K, or $NH_4$; and $R_2$ is H, -SCN, -Cl or -Br.

Preferably, $R_1$ is phosphate

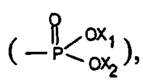

or succinic anhydride $[-C(=O)(CH_2)_4CO_2]$, in which $X_1$ and $X_2$ are Na, $R_2$ is -SCN.

The third type of novel derivatives of triptolide is as follow:

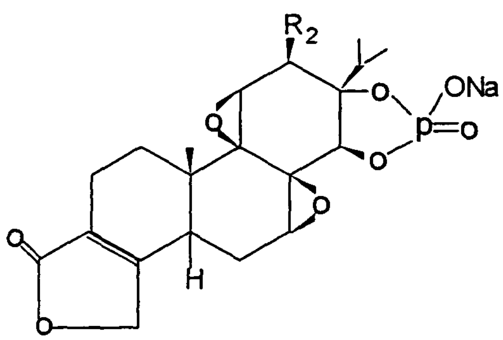

or

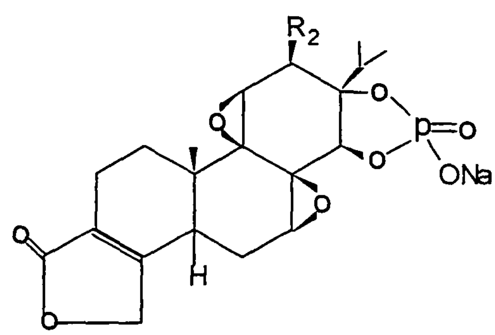

IIIa IIIb

wherein $R_2$ is H, -SCN, -Cl or -Br.

Another objective of the present invention is to provide the synthetic methods of these novel highly water-soluble derivatives of triptolide, and more particularly, the synthetic methods of preparing above three types of water-soluble derivatives of triptolide from the following two lead compounds (see Example 1-7 for details).

The first lead compound is triptolide:

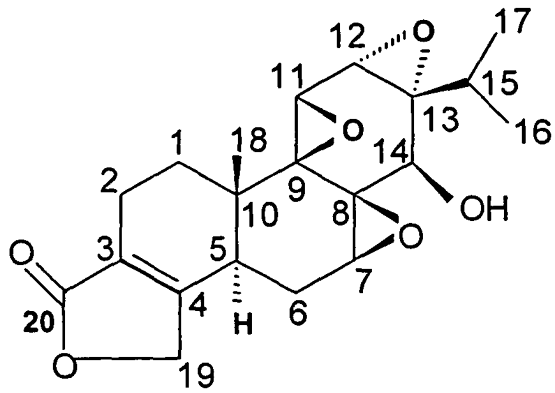

**Triptolide(T)**

US Patent 5,962,516 discloses a method for the preparation of 14-succinyl triptolide and sodium salt thereof from triptolide. 14-β-phosphate bisodium triptolide produced by phosphorylation according to the present invention, however, has higher water solubility.

The second lead compound is 12-β-thiocyano-13-α-hydroxy triptolide:

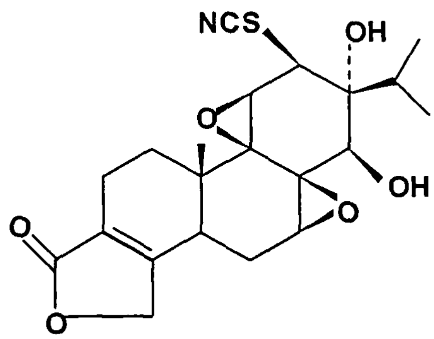

**12-βthiocyano-13-α-hydroxy triptolide (T-SCN)**

Another objective of the present invention is to provide molecular biological evidence of immunosuppressive activity of these novel highly water-soluble derivatives of triptolide (Example 8, 9). It has been demonstrated these novel derivatives of triptolide significantly inhibit the production of cytokines such as IL-1, IL-2, IL-6, iNOS and Cox-2.

A further objective of the present invention is to provide animal experiment evidence of low toxicity of these novel highly water-soluble derivatives of triptolide (Example 10). The water-soluble derivatives of triptolide prepared from T-SCN according to present invention have a significantly decreased toxicity. For example, the toxicity of sodium 12-β-thiocyano-triptolide-13-β-14-α-phosphate with a $LD_{50}$ of 126 mg/kg body weight is much lower than that of triptolide which has a $LD_{50}$ of 0.85 mg/kg body weight, while the former has a quite high immunosuppressive activity.

A still another objective of the present invention is to provide animal experiment evidence of treating autoimmune deficiency diseases with these novel highly water-soluble derivatives of triptolide. For example, the experiment of effect on DNCB-induced delayed hypersensitivity reaction of mice (Example 11) and anti-inflammatory test with cotton ball granulation in rat (Example 12) have demonstrated all of the novel WDY series derivatives of triptolide have significant immunosuppressive and anti-inflammatory activities.

## Description of the Figures

Fig 1. Effect of water-soluble derivatives of triptolide according to the present invention on IL-2 (medium: blank control; PHA: lipopolysaccharide, CsA: Cyclosporine A).
Fig 2. Effect of water-soluble derivatives of triptolide according to the present invention on IL-1, IL-6 and iNOS (medium: blank control; PHA: lipopolysaccharide, CsA: Cyclosporine A).
Fig 3. Effect of water-soluble derivatives of triptolide according to the present invention on $PGE_2$ content of HT-29 cell (pg/ml) (control = 22.93 pg/ml, indomethacin (indo) = 1.3 pg/ml, WDY 4 = 4.22 pg/ml, WDY 7 = 4.97 pg/ml).
Fig 4. The results of the effect of WDY 6 on contralateral paw.
Fig 5. The results of the effect of WDY 7 on contralateral paw.
Fig 6. The results of the effect of orally administrating WDY7 on the thickness of left paw.
Fig7. The results of the effect of orally administrating WDY6 on the thickness of left paw.

The present invention will be described in detail by the following Examples.

Example 1. Preparation of sodium 12-β-thiocyano triptolide-13-α-14-β-phosphate (WDY 1)

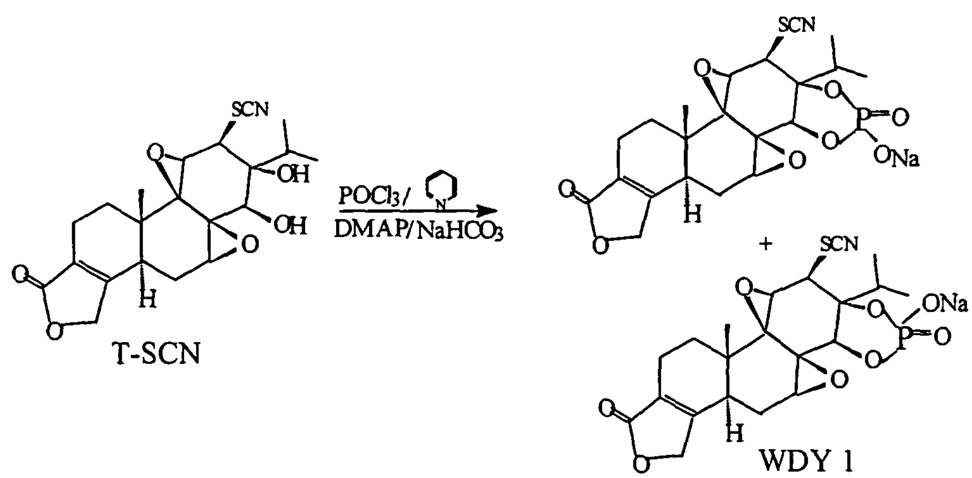


Under the protection of nitrogen atmosphere, 251 mg T-SCN (0.600 mmol), 20 ml pyridine, 0.28 ml $POCl_3$ (3.000 mmol) and 40 mg DMAP were sequentially added into a three-necked bottle. After sealing, the reaction mixture was stirred at room temperature for 24 hours, then hydrolyzed and neutralized to pH=8 with saturated aqueous $NaHCO_3$ in ice bath. The resulting mixture was concentrated under reduced pressure to dryness, and the residue was dissolved in acetonitrile and the inorganic salts were removed. TLC detection was conducted by developing with n-butanol: water: glacial acetic acid (10:1:1), and major point was product WDY1 comprising very little T-SCN and other by-products. The product was dissolved in acetonitrile and 2 g silica gel of 75-300 mesh was added. The acetonitrile was removed and the product WDY1 was purified by silica gel (75-300 mesh column chromatography using n-butanol (n-BuOH)/ water (20/1). The product was monitored and detected by TLC. The product points were collected and pooled, removed of solvent under reduced pressure and precipitated with ether. After standing for 1 hour and filtering, powder solid was obtained and dried with drying pistol (yield: 75%). Water solubility >100 mg/ml. $R_f$=0.24, (n-BuOH /$H_2O$/HAc = 10/1/1). Purple-red in developing reagent (Kedd's reagent).

MS ESI+ m/z: $C_{21}H_{23}NSO_8PNa$. Calculated value: 504.0858. Analytical value: 504.0857.
MS ESI- m/z: $C_{21}H_{23}NSO_8PNa$. Calculated value: 480.0877. Analytical value: 480.0888.
IR(KBr) $cm^{-1}$: 3417, 2938, 2156, 1749, 1674, 1247, 1109, 1000

$^1$HNMR,δppm: 0.84(3H, s, 18-$CH_3$), 1.01(3H, d, J=6Hz, 16-$CH_3$), 1.04(3H, s, 17-$CH_3$), 1.34(1H, m, 1-αH), 1.46 (1H, m, 1-βH), 1.80(lm, t, J=14.4Hz, 6- βH), 1.94(1H, m, 2-H), 2.13(1H, m, 2-H), 2.20(1H, m, 6-αH), 2.35(1H, m, 15-H), 3.09(1H, m, 5-H), 3.51(1H, br, 7-H), 3.71(1H, s, 12-H), 4.25(1H, br, 11-H), 4.61(1H, br, 14-H), 4.85(2H, dd, $J_1$=36Hz, $J_2$=17.2Hz, 19-H);

$^{13}$CNMR, δppm: 15.4(18-C), 17(2-C), 19.2(16-C), 20(17-C), 21.8(6-C), 29.5(1-C), 32.7(15-C), 35.0(10-C), 39(5-C), 55.7(11-C), 56.0,56.2(8-C), 56.7(7-C), 62.5(12-C), 66.0(9-C), 70.8(19-C), 77.5(14-C), 83.7(13-C), 112(SCN), 123(3-C), 163(4-C), 173.6(20-C).

$^{31}$PNMR, δppm: 4.57.

Example 2. Preparation of 12-β-thiocyano-13-α-hydroxy triptolide-14-β-succinic acid mono-ester (WDY6)

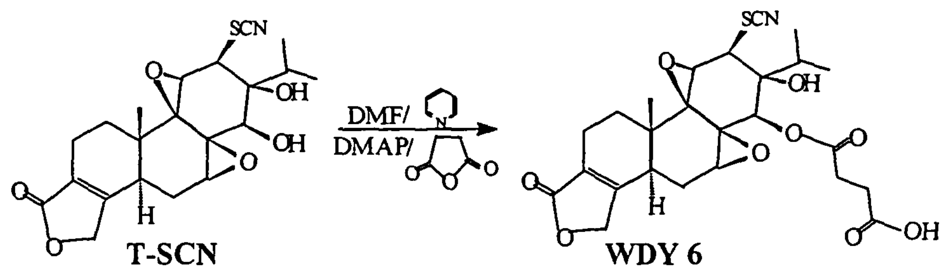


T-SCN WDY 6

80 mg 12-β-thiocyano-13-α-14-β-hydroxy triptolide (T-SCN)(0.191 mmol), 8 ml pyridine, 4 ml DMF, 24 mg DMAP and 3.2 g succinic anhydride (3.820 mmol) were sequentially added into a 50 ml three-necked bottle at room temperature. After sealing and being agitated for a week, the reaction mixture was poured into ice water and extracted with methylene dichloride several times. The methylene dichloride phases were pooled. TLC detection: chloroform: methol (10:1). The main point was WDY 6 together with very little unreacted 12-β-thiocyano-13-α-14-β-hydroxy triptolide. After concentrating under reduced pressure, the solvent was dried out. Purified product was obtained by H silica gel column chromatography (chloroform: methanol=14:1). yield = 90%. $R_f$: 0.30 Melting Point: 127-129°C . Water solubility > 30 mg/ml.

Element analysis: $C_{25}H_{29}SNO_9$. calculated value %:C, 57.79. H, 5.63. N, 2.70. Analytical value %: C, 57.66. H, 5.91. N, 3.05.

IR: 3435, 2970, 2152, 1745, 1673, 1157, 1022, 993.

$^1$HNMR,δ ppm: 0.79(3H, d, J=6.4Hz,16-$CH_3$), 0.89(3H, s, 18-$CH_3$), 0.97(3H, d, J=6.4Hz, 17-$CH_3$), 1.29(1H, m, 1-αH), 1.44(1H, m, 1-βH), 1.83(1m, t, J=14.4Hz, 6-βH), 1.95(1H, m, 15-H), 1.99(1H, m, 2-H), 2.15(1H, m, 2-H), 2.23 (1H, m, 6-αH), 2.46~2.51(4H, -$CH_2CH_2$-), 2.69(1H, m, 5-H), 3.52(1H, d, J=6.4Hz, 7-H), 3.96(1H, d, J=5.2Hz,12-H), 4.03(1H, d, J=6.03Hz, 11-H), 4.53(1H, s, 14-H), 4.84(2H, dd, $J_1$=42Hz, $J_2$=18.4Hz 19-H); 5.53(1H, s, 13-OH), 12(1H, br, -COOH).

$^{13}$CNMR, δ ppm: 14.3(18-C), 15.7(17-C), 16.3(16-C), 16.8(2-C), 22.2(6-C), 28.8-28.9(-CH2CH2-), 29.4(15-C), 30.1(1-C), 35.2(10-C), 39.5(5-C), 50.9(12-C), 57.7(11-C), 59.0(8-C),62.1(7-C), 67.1(9-C), 70.6(19-C), 74.2(14-C), 75.6 (13-C), 114.0(SCN), 123.5(3-C), 162.2(4-C), 170.8(-CO-),173.38~173.43(-COOH, 20-C).

**Example 3:** Preparation of disodium triptolide-14-β-phosphate (WDY 7)

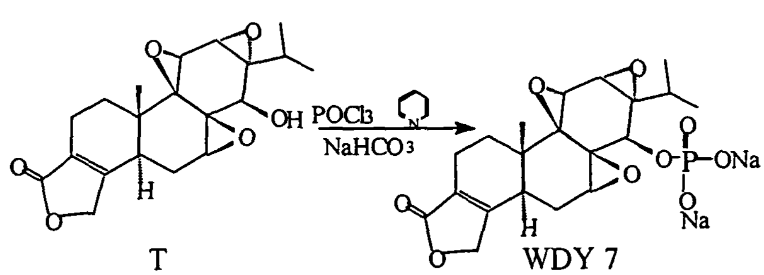


T WDY 7

Under the protection of nitrogen atmosphere, 180 mg T (0.500 mmol), 10 ml pyridine were added into a three-necked bottle, into which 0.14 ml $POCl_3$ (1.500 mmol) was then slowly added. After reacting for 2-3 hours under sealing, the reaction mixture was hydrolyzed and neutralized to pH=9 with saturated aqueous $NaHCO_3$ in ice bath. The resulting mixture was concentrated under reduced pressure to dryness, and the residue was dissolved in methanol and the

inorganic salts were removed. TLC detection was conducted by developing with n-butanol: water: glacial acetic acid (4:1:1), and major point was product WDY7 comprising very little T and other by-products. The product WDY7 was purified by silica gel column chromatography using n-butanol (n-BuOH) /water (15/2). The product was detected by TLC. The WDY7 was pooled, concentrated under reduced pressure to dryness, dissolved in tetrahydrofuran and precipitated with ether to obtain purified product (yield 167 mg, 75%). $R_f$=0.32. Water solubility >100 mg/ml.

MS:ESI+ m/z:$C_{20}H_{24}O_9PNa_2$ Calculated value:485.0956, Analytical value:485.0953.

IR(KBr)$cm^{-1}$: 3424, 2971, 2935, 2878, 1748, 1671, 1445, 1226, 1118, 1035, 975.

$^1$HNMR; δppm: 0.70 (3H, d, J=7.2Hz, 16-$CH_3$), 0.88(3H, d, J=6.8Hz, 17-$CH_3$), 0.96(3H, s, 18-$CH_3$), 1.25(1H, m, 1-αH), 1.30(1H, m, 1-βH), 1.82 (1H, t, J=13.8Hz, 6-βH), 1.96(1H, m, 2-H), 2.08(1H, m, 2-H), 2.13(1H, m, 6-αH), 2.21 (1H, m, 15-H), 2.55(1H, m, 5-H), 3.36(1H, m, 7-H), 3.83(1H, m, 11-H), 4.11(1H, d, J=11.6Hz, 14-H), 4.83(2H, dd, $J_1$=35.0Hz, $J_2$=17.4Hz, 19-H).

$^{13}$CNMR, δppm: 14.4(18-C), 17.1(2-C), 17.5(16-C), 18.0(17-C), 23.1(6-C), 26.0(15-C), 29.7(1-C), 35.7(10-C), 40.3(5-C), 54.7(12-C), 55.5(11-C), 60.8(7-C), 71.0(19-C), 75(14-C), 123.8(3-C), 163.2(4-C), 174.1(20-C), 61.1, 64.2, 65.2, 65.3(8-C, 9-C, 13-C).

**Example 4.** Preparation of sodium triptolide-14-β-phosphite (WDY 4)

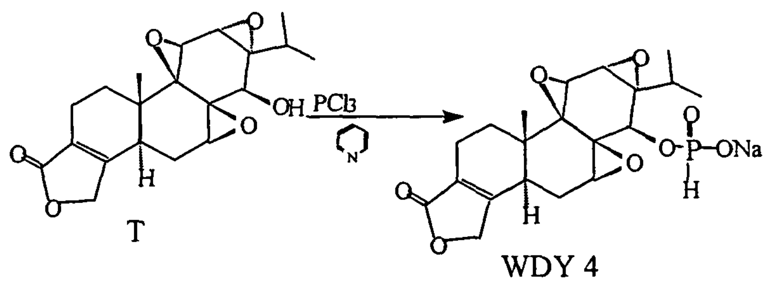


T

WDY 4

Under the protection of nitrogen atmosphere, 200 mg triptolide (0.556 mmol) and 20 ml pyridine were added into a three-necked bottle of 25ml, whereafter 0.10 ml $PCl_3$ (1.149 mmol) was slowly dropwise added. Upon feeding, nitrogen gas was stopped to enter the three-necked bottle. After reacting for 1 hour under sealing, the reaction bottle was cooled down in ice bath. Then saturated aqueous $NaHCO_3$ was slowly added to the reaction mixture to conduct hydrolytic reaction and neutralize to pH=9. The solvent in the mixture was vaporized to dryness under reduced pressure, and the residue was dissolved in chloroform/methanol (5/2) and the inorganic salts therein were removed. TLC detection was conducted by developing with n-butanol: water: glacial acetic acid (4:1:1), and major point was product WDY4 comprising a little other by-products. The product WDY4 was purified by H silica gel column chromatography using chloroform/methanol (5/2). The product was detected by TLC. The WDY4 was pooled, concentrated under reduced pressure to remove the solvent, dissolved in eluent and precipitated with ether to obtain purified product (yield 70 mg, 50%). $R_f$=0.46. Purple-red in developing reagent (Kedd's reagent). Water solubility >100 mg/ml.

IR(KBr)$cm^{-1}$: 3424, 2965, 2365, 1750, 1627, 1226, 1028, 972.

$^1$HNMR,δppm: 0.74(3H, d, J=7.2Hz, 16-$CH_3$), 0.90(3H, d, J= 6.8Hz, 17-$CH_3$), 0.94(3H, s, 18- $CH_3$), 1.25(1H, m, 1-αH), 1.32(1H, m, 1-βH), 1.80(1H, t, J=14.2Hz, 6- βH), 1.94(1H, m, 2-H), 2.10(1H, m, 2- H), 2.20(1H, m, 6-αH), 2.33 (1H, m, 15- H), 2.59(1H, m, 5-H), 3.29(1H, m, 7-H), 3.52(1H, d, J=3.2Hz, 11-H), 3.82(1H, d, J=3.2Hz, 12-H), 4.03(1H, d, J=12.4Hz, 14-H), 4.83(2H, m, 19-H), 6.7(1H, d, J=595.6Hz, P-H).

$^{13}$CNMR, δppm: 13.9(18-C), 16.7(2-C), 17.0(16-C), 17.5(17-C), 22.8(6-C), 26.3(15-C), 29.2(1-C), 35.3(10-C), 40.1(5-C), 54. 3(12-C), 54.9(11-C), 60.4(7-C), 70.3(19-C), 73.(C-14, d, J=22.8Hz), 123.2(3-C), 162.5(4-C), 173.2 (20-C), 63.5, 64.5, 64.6(8-C, 9-C, 13-C).

$^{31}$PNMR, δppm: 1.34(d, $J_{P-H}$=599Hz).

**Example 5.** Preparation of sodium 12-β-chlorotriptolide-13-α-14-β-phosphate (WDY 2)

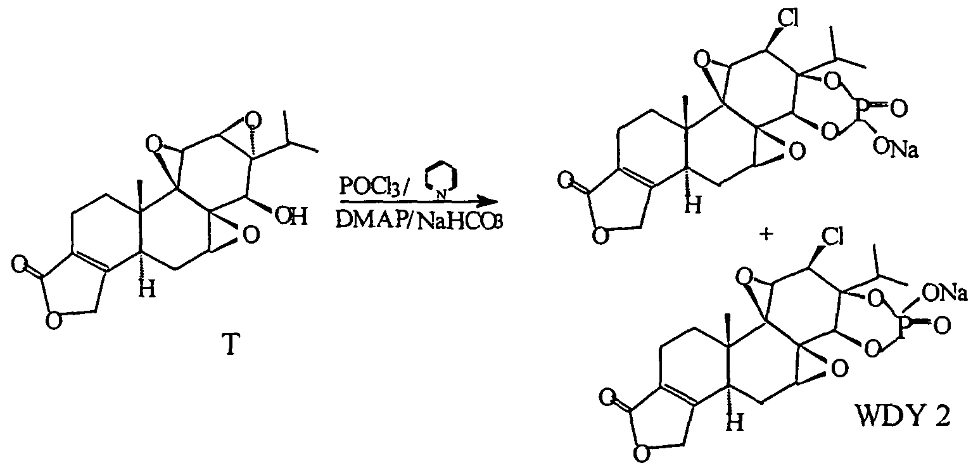


Under the protection of nitrogen atmosphere, 108 mg triptolide (0.3 mmol), 30 ml pyridine and 39 mg DMAP were added into a three-necked bottle, whereafter 1.5 ml $POCl_3$ (16.37 mmol) was dropwise added. Upon feeding, nitrogen gas was stopped to enter the three-necked bottle. After reacting for 24 hour at room temperature under sealing, the reaction bottle was cooled down in ice bath. Then saturated aqueous $NaHCO_3$ was slowly added to the reaction mixture to conduct hydrolytic reaction and neutralize to pH=8. The solvent in the mixture was vaporized to dryness under reduced pressure, and the residue was dissolved in tetrahydrofuran and the inorganic salts therein were removed. TLC detection was conducted by developing with n-butanol: water: glacial acetic acid (10:1:1), and major point was product WDY2 comprising very little T and other by-products. The product WDY2 was purified by silica gel column chromatography using n-butanol/water (15/2). The product was detected by TLC. The WDY2 was pooled, concentrated under reduced pressure to remove the solvent, dissolved in a small amount of tetrahydrofuran and precipitated with ether to obtain purified product (yield 115.2 mg, 80%). $R_f$=0.33. Purple-red in developing reagent (Kedd's reagent). Water solubility >100 mg/ml.

Element analysis: $C_{20}H_{23}O_7ClPNa$ Calculated value %: C 49.96, H 4.82, Analytical value %: C 49.75, H 4.43.

IR(KBr)$cm^{-1}$: 3428, 2930, 1741, 1634, 1244, 1023, 582

$^1$HNMR,δppm: 0.78 (3H, d, J=6.8Hz, 16-$CH_3$), 0.96 (3H, d, J= 6.8Hz, 17-$CH_3$), 1.0 (3H, s, 18-$CH_3$), 1.36 (1H, m, 1-αH), 1.38 (1H, m, 1-βH), 1.89 (1H, m, 6-βH), 2.06(1H, m, 2-H), 2.16 (1H, m, 15-H), 2.21 (1H, m, 2-H), 2.39 (1H, m, 6-αH), 3.05 (1H, d, J=12.8Hz, 5-H), 3.54(1H, d, J=2.4Hz, 12-H), 3.86 (1H, d, J=2.8Hz, 11-H), 4.44(1H, s, 14-H), 4.63 (1H, s, 7-H), 4.83 (2H, m, 19-H).

$^{13}$CNMR, δppm: 15.0(18-C), 16.8(16-C), 17.6 (2-C), 18.0 (17-C), 28.5 (6-C), 28.9 (15-C), 29.9 (1-C), 37.0 (10-C), 37.5 (5-C), 53.4 (12-C), 57.8 (11-C), 63.7 (7-C), 70.7 (19-C), 74.6 (14-C), 124.0 (3-C), 163.3 (4-C), 173.5 (20-C), 61.8, 61.9, 62.9, 81.4 (8-C, 9-C, 13-C).

$^{31}$PNMR, δppm: 2.96.

**Example 6.** Preparation of disodium 12-β-thiocyano-13-α-hydroxy triptolide-14-β-phosphate (WDY3)

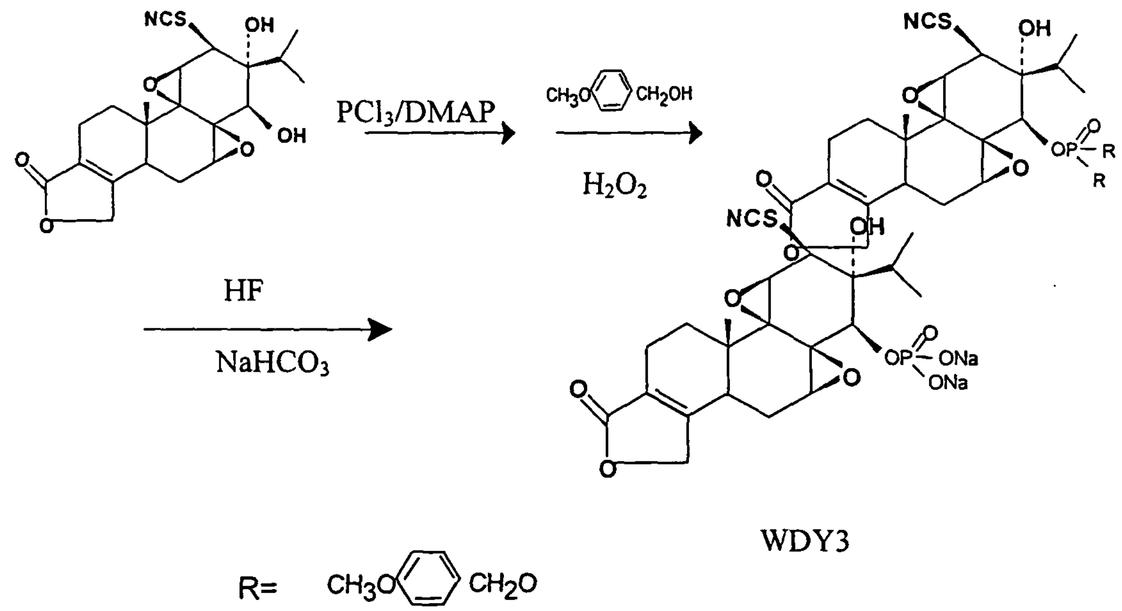


WDY3

Under the protection of nitrogen atmosphere, 41.9 mg T-SCN (0.100 mmol), 5 ml pyridine, 8 mg DMAP and 0.018 ml PCl3 (0.200 mmol) were sequentially added into a three-necked bottle. Upon sealing, the reaction mixture was agitated at room temperature and 0.065 ml (0.520 mmol) p-methoxybenzyl alcohol was added. After reacting at room temperature for 10 min, the reaction mixture was added 0.200 ml 30% $H_2O_2$. After another 30 min, the mixture was cooled down in ice bath and added saturated aqueous $NaHCO_3$ to conduct hydrolytic reaction. The solvent in the mixture was vaporized to dryness under reduced pressure, and the residue was dissolved in ethyl acetate and the inorganic salts therein were removed. After washing with saturated aqueous NaCl solution for several times and drying over anhydrous $Na_2SO_4$, the residue was concentrated under reduced pressure to dryness and dissolved in acetonitrile. Then 2 g silica gel of 75-300 mesh was added. The acetonitrile was removed completely and the product was purified by silica gel (75-300 meshcolumn chromatography using n-butanol (n-BuOH) /water (15/2). The product was monitored and detected by TLC. The product points were collected and pooled, removed of solvent under reduced pressure and precipitated with ether. After standing for 1 hour and filtering, powder solid was obtained and dried with drying pistol. $R_f$=0.24, (n-BuOH /$H_2O$/HAc = 4/1/1). Purple-red in developing reagent (Kedd's reagent). Water solubility >100 mg/ml.

**Example 7.** Preparation of sodium 12-β-thiocyano-13-α-hydroxy triptolide-14-β-phosphite and sodium 12-β-thiocyano-14-β-hydroxy triptolide-13-α-phosphite (WDY5)

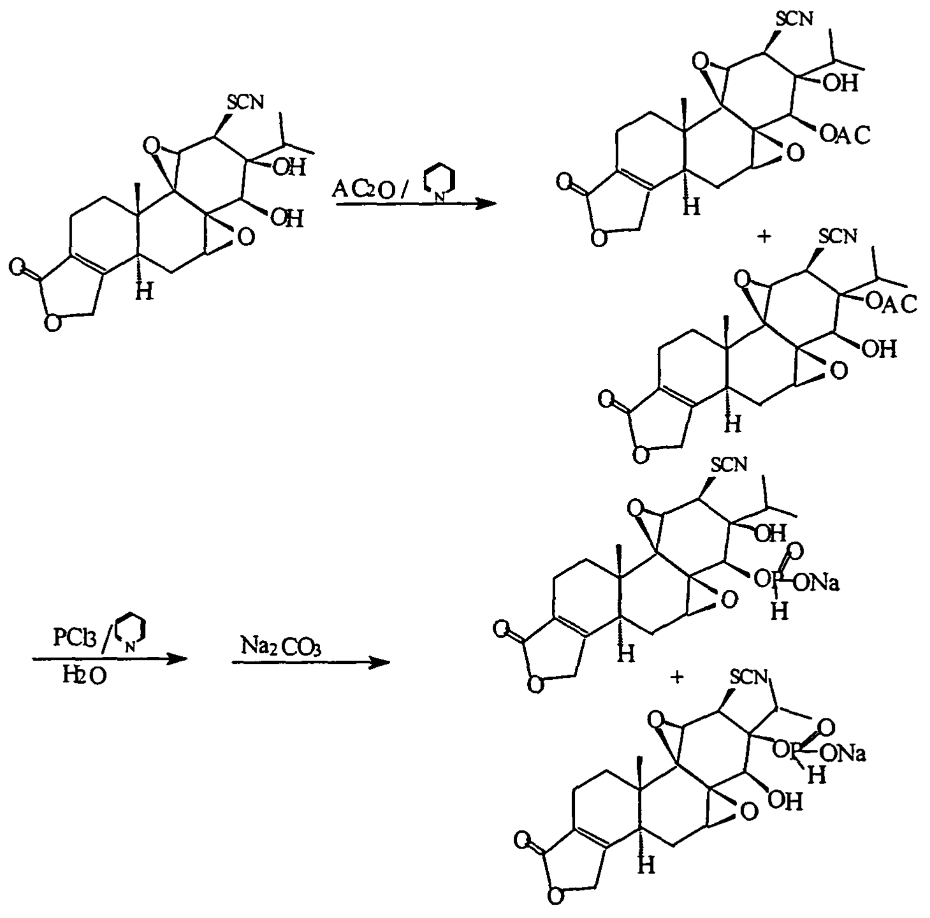


WDY 5

80 mg T-SCN (0.191 mmol) and 8 ml pyridine were added into and dissolved in a three-necked bottle with agitation, into which 8 ml acetic anhydride was further added. The reaction mixture was stirred at room temperature for one week under sealing, and then stirred at 40°C for 10 hours. Upon completion, the reaction mixture was poured into ice water and extracted three times with methylene dichloride. The combined solution of methylene dichloride phases was washed three times with saturated aqueous $NaHCO_3$ and with saturated aqueous NaCl to neutrality. The solution was dried over anhydrous sulfate sodium for 24 hours, and concentrated under reduced pressure to remove the solvent. The crude product was detected by TLC. TLC detection was conducted by developing with chloroform: methanol (10:1), and major point was acetylated T-SCN comprising a small amount of T-SCN. The product was dissolved in acetone and 0.5 g silica gel was added and stirred. Silica gel column chromatography was conducted using chloroform : methanol (15:1) as eluant. The product was detected by TLC. The acetylated T-SCN fractions were pooled and 70 mg product was obtained. Under the protection of nitrogen atmosphere, 70 mg acetylated T-SCN was dissolved in 2 ml pyridine in a three-necked bottle and 0.1 ml $PCl_3$ was further added. The mixture was stirred at room temperature for 30 min under sealing. The reaction mixture was cooled down in ice bath, hydrolyzed and 40 ml methylene dichloride was added. After washing with water twice, 60 ml of water phases were combined and detected by TLC. TLC detection was conducted by developing with n-butanol: water: glacial acetic acid (4:1:1), and almost no impurity was found. To above 60 ml aqueous solution was added 40 ml saturated aqueous $Na_2CO_3$ and hydrolyzed at room temperature for 18 hours. The resulting solution was neutralized to pH=7 with dilute $H_2SO_4$, distilled under reduced pressure to remove water and desalted with ethanol. The obtained product was detected by TLC (n-butanol: water: glacial acetic acid (10: 1:1)). The product point was obvious, but there were many impurity points. WDY5 was obtained by silica gel column chromatography using $CHCl_3/CH_3OH$ (4/1). $R_f$=0.34. Water solubility >100 mg/ml.

**Example 8.** Effects of WDY compounds on cytokines IL-1, IL-2, and IL-6, and iNOS

Cytokines, prostaglandins and nitric oxide are important mediators of the immune system and are expressed in various kinds of organisms and cells associated with autoimmune deficiency diseases and inflammatory diseases. To study the inhibitory effects of drugs on the production of these inflammatory mediators, the quantitative real-time reverse transcriptase-polymerase chain reaction (RT-PCR) assays were used to quantitate the mRNA levels of IL-1, IL-2, IL-6, and iNOS mRNA expressed in rat spleen lymphocytes stimulated with LPS or PHA. Both cyclosporin A and compound WDY-4 had completely inhibited LPS- or PHA-induced IL-2 mRNA expression (Fig. 1). In addition, WDY-4 inhibited the mRNA expression of IL-6 and iNOS completely (Fig. 2), while cyclosporin A had a weak effect on these two cytokines. Besides, the high immunosuppressive activity and very low toxicity of WDY1 and WDY6 had been demonstrated by the results of animal experiments, suggesting the efficacy of the derivatives according to the present invention in the treatment of autoimmune deficiency diseases and inflammatory diseases.

**Experimental Methods**

Splenocytes were obtained from Lewiz rats according to standard protocol and seeded in a 6-well plate with a cell density of $1x10^6$/ml. The cell culture was added with compounds at an appropriate concentration and preincubated at 37°C for 15 min. Then 1 µg/ml of LPS (Sigma, St. Louis, MO) or 1 µg/ml PHA (Sigma, St. Louis, MO) were added and incubated at 37°C in a humidified 5% $CO_2$ incubator for 2 hours. Total RNA were extracted from the cultured splenocytes using the Qiagen RNA Preparation Kit (Qiagen).

RT-PCR experiments were carried out according to the instructions of the ABI TaqMan Analysis Kit provided by the manufacturer (Perkin Elmer Applied Biosystems, Foster, CA). Total RNA (1 µg) of rat splenocytes were reverse transcribed to complementary DNA (cDNA) by a reaction system containing 2 mM deoxynucleotide mixture, 100 mM DTT, 40 units of RNase inhibitor, 50 ng random primers, and 15 units of Thermoscript reverse transcriptase. The reaction condition was as follows: 25°C for 10 min and 48°C for 45 min, then 90°C for 5 min, and finally cooled down to 4°C. The expression levels of IL-1, IL-2, IL-6, iNOS, and cyclophilin were determined by TaqMan PCR technology using the ABI 7700 Sequence Detector (Perkin Elmer Applied Biosystems). The reaction system was as follows: 1x TaqMan Universal PCR Master Mix, 900 nM of forward (upstream) and reverse (downstream) primers and 200 nM TaqMan probe, 50 nM primers and probes. The thermal cycling condition was as follows: 95°C for 15 min and 60° C for 1 min for 40 cycles. The production of mRNA of interest and that of mRNA of cyclophilin were calculated with correlation standard curve. The results were shown in figures 1 and 2.

**Example 9.** Detection of the inhibition of WDY on Cox-2 by $PGE_2$ enzymatic assay

The effect of WDYs on the production of $PGE_2$ from arachidonic acid (AA), which was catalyzed by COX-2, was detected by $PGE_2$ EIA Kit (Cayman Company). HT-29 human colon cancer cells with an appropriate density were seeded in PRMI 1640 medium containing 10% fetal calf serum and suitable amounts of penicillin and streptomycin. The next day, the medium was replaced with fresh medium after cell adhesion and added with WDY4 or WDY7 (10 µg/ml). Indomethacin (100 µg/ml) was used as positive control and the sample without drugs was used as negative control. After incubation with drugs for 24 hours, the medium was removed. The cells were washed with PBS and added into 1 ml serum-free RPMI 1640 medium containing 40 µM arachidonic acid (AA). After incubation at 37°C for 30 min, the supernatant was collected and subjected to derivation overnight according to the instructions of the Kit. The production of $PGE_2$ was detected and calculated and the results were shown in figure 3.

**Example 10.** The $LD_{50}$ of i.v. administration of WDY1

WDY 1 was dissolved in water and divided into five dose groups (150, 135, 122, 109, 98 mg/kg body weight). 10 Kunming mice (Shiuchuan Experimental Animal Center No. 99-30) consisting of 5 females and 5 males in each group were used to determine the acute toxicity $LD_{50}$ of WDY 1, which was injected via caudal vein. The acute toxicity LDso of WDY1 was 126 mg/kg body weight.

Table 1.

| Acute toxicity $LD_{50}$ of WDY 1 | | | | | | |
|---|---|---|---|---|---|---|
| Dose (mg/kg) | Log (D) | No. of mice | Death No. of mice | Mortality (%) | $LD_{50}$ | 95% limit |
| 150 | 2.1761 | 10 | 9 | 90 | 126 | 119-133 |
| 135 | 2.1303 | 10 | 8 | 80 | | |
| 122 | 2.0864 | 10 | 4 | 40 | | |
| 109 | 2.0374 | 10 | 1 | 10 | | |
| 98 | 1.9912 | 10 | 0 | 0 | | |
| Y=38.1841+20.56654 log (D) (G=0.2063235 | | | | | | |

**Example 11.** Effects of WDYs on DNCB-induced delayed hypersensitivity reaction

Experimental principle: Dinitrochlorobenzene (DNCB) is a hapten. When being applied onto abdomen skin, it combines with skin proteins to form a complete antigen and thereby stimulates the TLC to proliferate into sensitized lymphocytes. 4-7 days later, DNCB is again applied onto the skin and results in topical delayed allergic reaction (edema). The reaction generally reaches a peak 24-48 hours after the antigen attack, at which time local swelling is examined.

Evaluation: The effects of drugs on cellular immunity were detected. In addition, cellular immunity enhancing model and cellular immunity decreasing model were used. Delayed hypersensitivity reaction is a tissue damage occurring 24-48 hours after the antigen attack in sensitized body. The strength of delayed hypersensitivity reaction may be represented as the degree of swelling of body

1. Experimental materials:

Test Drugs: WDY 1, WDY 6 and WDY 7. All of them were white powder and formulated into desired concentration with physiological saline just before use.
Animal: 110 Kunming mice from Sichuan Experimental Animal Center, Lot No. 99-30 were used for the experiment.
Reagents: 2,4-dinitrochlorobenzene (DNCB), Lot No. 20000101, manufactured by Shanghai First Reagent Factory. They were formulated into the following concentrations with acetone before use: 50%, 2%, 0.5% and 0.15%.

2. Experimental method: The experiment was designed according to the *Guideline of Pre-clinical Studies for Efficacy of Anti-Inflammatory and Immunoregulatory Drugs* published by the Chinese Ministry of Public Health.

Dose: The grouping and administration were shown in Table 1.

Table 2.

| Treatment groups | | | | |
|---|---|---|---|---|
| Group | Dose (mg/kg) | Concentration (mg/ml) | dosextimes of administration (ml/10g×times) | Administration pathway |
| Model group | NS | - | 0.1×10 | ip. |
| WDY1 low<br>WDY1 middle<br>WDY1 high | 2.4<br>6<br>15 | 0.24<br>0.6<br>1.5 | 0.1×10<br>0.1×10<br>0.1×10 | ip.<br>ip.<br>ip. |
| WDY6 low<br>WDY6 middle<br>WDY6 high | 3.2<br>8<br>20 | 0.32<br>0.8<br>2 | 0.1×10<br>0.1×10<br>0.1×10 | ip.<br>ip.<br>ip. |
| WDY7 low | 0.05 | 0.005 | 0.1×5 | ip. |

Table 2. (continued)

| Treatment groups | | | | |
|---|---|---|---|---|
| Group | Dose (mg/kg) | Concentration (mg/ml) | dosextimes of administration (ml/ 10g×times) | Administration pathway |
| WDY7 middle | 0.14 | 0.014 | 0.1×5 | ip. |
| WDY7 high | 0.4 | 0.04 | 0.1×5 | ip. |
| Cyclosporin-A | 100 | 10 | 0.1×10 | po. |

Sensitization: Back hair was removed from a patch of skin of each mouse. By using a microsyringe, 2 μl of 50% DNCB in acetone was dropped on the naked skin to sensitize animal.

Treatment: The mice were administrated according to above table staring from the day of sensitization, once a day for ten days (WDY7 was administrated every other day).

Provocation: At the tenth day after administration, hair was removed from the abdomen of each mouse at three sites with a size of 1 cm. Then 20 μl of 2%, 0.5% and 0.15% of DNCB solutions in acetone were dropped onto the naked skin at three sites to provocate animal, respectively.

Evaluation Standards: 24 hrs, 48 hrs, and 72 hrs after the provocation, the reactions of skins at the three sites were observed and scored according the following table. The sum of scores at the three sites was taken as the evaluation standard and compared with control group.

Table 2.

| Evaluation Standard of the strength of skin reaction | |
|---|---|
| Grade (Score) | Skin reaction |
| 0 | No reaction |
| 0.5 | Slight color change, or rash |
| 1.0 | Marked change in color (yellow or red), but no swell or oedema |
| 2.0 | Marked change in color (yellow or red), with swell or oedema |
| 3.0 | Swelling and slight necrosis |
| 4.0 | Necrosis, scab |

3. Experimental results

Table 3

| Group | Time after Provocation | | |
|---|---|---|---|
| | 24h | 48h | 72h |
| Model group | 2.4±0.84 | 3.30±0.71 | 3.30±1.3 |
| Positive control (Cyclosporin A) | 0.6±0.21** | 0.85±0.34** | 1.55±0.50** |
| WDY1 low | 0.55±0.37** | 0.90±0.51** | 1.25±0.75** |
| WDY1 middle | 0.80±0.79** | 1.15±1.11** | 1.50±0.97** |
| WDY1 high | 0.30±0.26** | 0.70±0.26** | 0.85±0.24** |
| WDY6 low | 0.45±0.37** | 0.40±0.32** | 0.55±0.37** |
| WDY6 middle | 0.50±0.58** | 0.85±0.47** | 0.83±0.25** |
| WDY6 high | 0.25±0.26 | 0.55±0.37** | 0.55±0.28** |
| WDY7 low | 0.40±0.21** | 0.40±0.21** | 0.75±0.26** |
| WDY7 middle | 0.20±0.26** | 0.41±0.21** | 0.65±0.34** |
| WDY7 high | 0.0±0.0** | 0.17±0.29** | 0.17±0.29** |

**Example 12.** Anti-inflammatory test of $WDY_1$, $WDY_6$, $WDY_7$ --- cotton ball granulation

Experimental principle: A cotton ball placed into the rat body can cause connective tissue proliferation. This granulation proliferation is similar to later pathological changes caused by some clinic inflammatory diseases. Therefore this is used for the evaluation of effects of a drug against connective tissue proliferation. The comparison of granulation weights between drug treatment group and negative control group helps to evaluate a drug's anti-inflammatory efficacy. If there is significant difference between the two groups, the drug is considered to have anti-inflammatory effect in such inflammation model. The positive control drug is hydrocordisone.

1. Experimental materials: 110 male Wister rats of 200-250 g. Test drugs: WDY1, WDY 6 and WDY 7. Positive Control: Hydrocordisone.
2. Experimental method: The experiment was designed according to the *Guideline of Pre-clinical Studies for Efficacy of Anti-Inflammatory and Immunoregulatory Drugs* published by the Chinese Ministry of Public Health.

110 rats were anaesthetized (i.p.) with 30 mg/kg pentobarbital sodium, and then implanted 20 mg of sterilized dry cotton ball on the right side of abdomen. The next day, the rats were grouped and administrated according to the table 1. 24 hours after the last administration, rats were sacrificed and the cotton balls were taken out. Fat tissue was shaved off from the cotton balls, which were then baked in an oven at 60°C for 1 hour. Thereafter, the weights of the cotton balls were recorded. 20 mg (original weight of cotton ball) was subtracted from each measured weight value of cotton balls and the finial weight value was used to evaluate the degree of swelling and represented as mg/100 g body weight. The data of drug treatment group was compared with those of control group.

3. Experimental results:

Table 4:

| Anti-inflammatory Effects of WDY1, $WDY_6$ and $WDY_7$ in cotton granulation tests | | | | | |
|---|---|---|---|---|---|
| dosage × times | group | Administration pathway | No. of mice | Average granulation ($\bar{X}$ ±SD, conversed into mg/100g) | Inhibition of swelling(%) |
| -- | control | iv | 9 | 68.0±39.9 | |
| 40mg/kg×8 | Hydrocordisone | Po | 9 | 39.3±22.2 | 42.2 |
| 2.5mg/kg×8 | $WDY_1$ | iv | 8 | 36.5±11.2 | 46.3 |
| 5mg/kg×8 | $WDY_1$ | iv | 9 | 36.9±9.9 | 45.7 |
| 10mg/kg×8 | $WDY_1$ | iv | 9 | 27.7±6.2 | 59.3 |
| 2.5mg/kg×8 | $WDY_6$ | iv | 8 | 39.0±18.2 | 42.6 |
| 5mg/kg×8 | $WDY_6$ | iv | 9 | 36.0±11.8 | 47.1 |
| 10mg/kg×8 | $WDY_6$ | iv | 8 | 34.7±10.2 | 49.0 |
| 0.025mg/kg×4 | $WDY_7$ | Iv (every other day) | 8 | 39.8±9.5 | 41.5 |
| 0.06mg/kg×4 | $WDY_7$ | Iv (every other day) | 8 | 30.2±4.6 | 55.6 |
| 0.125mg/kg×4 | $WDY_7$ | Iv (every other day) | 9 | 29.4±6.1 | 56.8 |

As shown in Table 4, the hydrocordisone group showed a significantly decreased weight of cotton ball granulation compared with control group. All of $WDY_1$, $WDY_6$ and $WDY_7$ showed significant effect against cotton ball granulation. Compared with the control group, high and middle dosages of three samples and low dosage of $WDY_1$ showed significant decrease in terms of the size of cotton ball granulation. Furthermore, there is a positive dose-effect relationship.

Example 13: Acute toxicity test

The $LD_{50}$ of i.v. administration of WDY6

A group of Kunming mice (Shiuchuan Experimental Animal Center No. 99-30) consisting of 5 females and 5 males was used to determine the acute toxicity $LD_{50}$ of WDY 6. WDY 6 was dissolved in physiological saline and divided into five dose groups (215, 187, 163, 142, 123 mg/kg body weight). The WDY 6 was injected via caudal vein and its acute toxicity $LD_{50}$ was determined to be 172 mg/kg body weight.

Table 5.

| Acute toxicity $LD_{50}$ of WDY 6 | | | | | |
|---|---|---|---|---|---|
| Dose (mg/kg) | No. of mice | Death No. of mice | Mortality (%) | $LD_{50}$ | 95% limit |
| 215 | 10 | 9 | 90 | 172 | 156-189 |
| 187 | 10 | 8 | 70 | | |
| 163 | 10 | 4 | 40 | | |
| 142 | 10 | 2 | 20 | | |
| 123 | 10 | 0 | 0 | | |

With the same method, the acute toxicity $LD_{50}$ of WDY1 and WDY7 injected via caudal vein were determined to be 126 mg/kg body weight and 0.8 mg/kg body weight, respectively.

Example 14: Effects of i.v. administration of WDY 6 and WDY 7 in the treatment of rat adjuvant-induced arthritis model

Experimental method

A rat adjuvant-induced arthritis model was constructed according to the method described by Perper RJ et al. (The use of a standardized adjuvant arthritis assay to differentiate between anti-inflammatory and immunosuppressive agents. Proc Soc Exp Biol Med. 1971, 137:506-512) and used to evaluate the effects of WDY 6 and WDY 7 in the treatment of rat adjuvant-induced arthritis. The swelling of hind paw was measured with the thickness of paw.

64 male SD rats (Shiuchuan Experimental Animal Center No. 99-32) were randomly divided into 8 groups. WDY 6 and WDY 7 were dissolved in physiological saline and divided into five dose groups, respectively. WDY 6 and WDY 7 were injected via caudal vein. Physiological saline was used as blank control and 30 mg/kg body weight of hydrocordisone was used as positive control.

Table 6

| dosages and treatment method (iv) | | | | |
|---|---|---|---|---|
| group | Dose mg/kg body weight | Concentration mg/ml | Times of treatment | Injection amount Ml/ 100g body weight |
| Control | - | - | 8 | 0.2 |
| WDY6 | 2.5 | 1.25 | 8 | 0.2 |
| WDY6 | 5 | 2.5 | 8 | 0.2 |
| WDY6 | 10 | 5 | 8 | 0.2 |
| WDY7 | 0.025 | 0.0125 | 4 | 0.2 |
| WDY7 | 0.06 | 0.03 | 4 | 0.2 |
| WDY7 | 0.125 | 0.0625 | 4 | 0.2 |
| hydrocordisone | 30 | 15 | 8 | 0.2 |

The arthritis model was successfully constructed after treating with Freund's adjuvant for 18 days. The swelling of injected paw (right hind paw) was primary disease and the swelling of contralateral paw (left hind paw) was secondary disease. The secondary disease was related with immunity.

Experimental results:

Results of the effect of WDY6 on contralateral paw (left paw) were shown in figure 4. WDY6 significantly decreased the thickness of the contralateral paw and the decrease of thickness showed a dose-effect relationship. The effect of WDY6 was comparable with hydrocordisone. WDY6 significantly decreased the thickness of the contralateral paw even at low dosage (5 mg/kg body weight). Acute toxicity $LD_{50}$ of WDY 6 was 172 mg/kg and its safety coefficient "$LD_{50}$/effective dose" was 34 (172/5) - 68 (172/2.5). The above results demonstrated WDY6 was effective and safe.

Similar results were obtained for WDY7 (see fig. 5). The safety coefficient "$LD_{50}$/effective dose" of WDY7 was 32 (0.8/0.025).

Example 15: Effects of per oral administration of WDY 6 and WDY 7 in the treatment of rat adjuvant-induced arthritis model

Experimental method: Same as Example 14, except that i.v. administration was replaced by oral administration.

Table 7.

| Dosages and times of administration | | |
|---|---|---|
| Group | Dose mg/kg body weight | Times of oral administration |
| Control | - | 10 |
| WDY6 | 5 | 10 |
| WDY6 | 15 | 10 |
| WDY6 | 45 | 10 |
| WDY7 | 0.02 | 10 |
| WDY7 | 0.067 | 10 |
| WDY7 | 0.2 | 10 |
| hydrocordisone | 30 | 10 |

The experimental results (see fig. 6 & 7) demonstrated the oral administration of WDY6 or WDY7 had significant effect in the treatment of adjuvant-induced rat arthritis.

Example 16. The effects of WDY6 & WDY7 on the prolongation of survival time of transplanted heart in mouse allogeneic atrium graft experiment

Experimental method

Intact heart of six days old C57BL mouse (male or female) was grafted into the auricle of male balb/c mouse (body weight: 20-25 g) (Shiuchuan Experimental Animal Center No. 99-31). The beating of grafted heart was recorded by cardiogram or directly under anatomical lens. The stopping of heart suggested the occurring of rejection.

Dosage

WDY: 20, 10 and 5 mg/kg; administration daily, i.v. injection.
WDY: 0.25, 0.12 and 0.05 mg/kg; administration every other day, i.v. injection.
Cyclosporine A: 25 mg/kg; administration daily, oral administration.

Results:

Table 8.

| The effect of WDY6 & WDY7 on the survival time of transplanted heart | | | | | | | | |
|---|---|---|---|---|---|---|---|---|
| group | Number of amimal | survival time (days) | | | | | | Average survival time (days) |
| Control | 16 | 9 | 11 | 9 | 9 | 9 | 11 | 9.7±0.80 |
| | | 9 | 9 | 11 | 9 | 10 | 10 | |
| | | 10 | 9 | 10 | 10 | | | |
| WDY6 20mg/kg | 6 | 13 | 13 | 12 | 12 | 13 | 13 | 12.7±0.52** |
| WDY6 10mg/kg | 8 | 13 | 13 | 12 | 13 | 12 | 12 | 11.9±1.2** |
| | | 10 | 10 | | | | | |
| WDY6 5mg/kg | 6 | 10 | 9 | 8 | 12 | 10 | 11 | 10.0±1.4** |
| WDY7 0.25mg/kg | 6 | 14 | 15 | 13 | 14 | 13 | 14 | 13.8±0.75** |
| WDY7 0.12mg/kg | 8 | 14 | 11 | 12 | 12 | 13 | 12 | 11.9±1.5** |
| | | 11 | 9 | | | | | |
| WDY7 0.05mg/kg | 6 | 11 | 15 | 9 | 11 | 11 | 12 | 11.5±2.0* |
| Cyclosporine A 25 mg/kg | 8 | 17 | 16 | 15 | 15 | 18 | 15 | 16.1±1.1* |
| | | 16 | 17 | | | | | |

The average survival times of grafted hearts in mouse allogeneic atrium graft experiments using WDY6, WDY7 and Cyclosporine A were 12.7, 13.8 and 16.1 days, respectively. The average survival time of control group was only 9.7 days. The above results demonstrated both WDY 6 and WDY7 had significant anti-rejection effect. The mice treated with WDY6 and WDY7 did not show a decrease of body weight and their appetites were normal, while those treated with Cyclosporine A showed a decrease both in body weight and appetite.

## Claims

**1.** A triptolide derivative of formula I

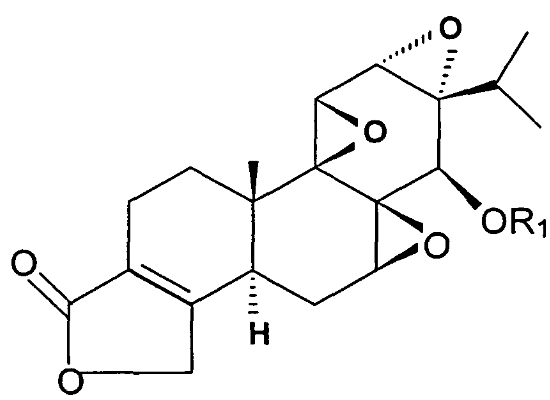

I

wherein $R_1$ is H, phosphate

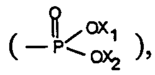

or phosphite

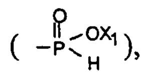

and $X_1$ and $X_2$ are Na, K, or $NH_4$.

**2.** A method for preparation of the compound according to claim 1, comprising the step of reacting the first lead compound triptolide with $POCl_3$, $PCl_3$ or other phosphonate halide, phosphate, phosphite halide, phosphite.

**3.** A triptolide derivative of formula II

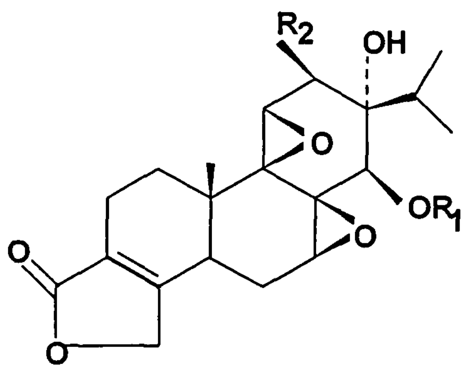

II

wherein $R_1$ is H, alkyl having 1-4 carbon atom(s), $-C(=O)(CH_2)_nCO_2$ , wherein n is an integer of 1-4, or phosphate

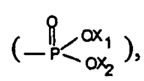

or phosphite

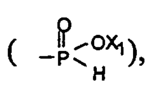

wherein $X_1$ and $X_2$ are Na, K, or $NH_4$; and $R_2$ is H, -SCN, -Cl or -Br.

**4.** A method for preparation of the compound according to claim 3, comprising the step of esterifying or phosphoric esterifying the second lead compound 12-β-thiocyano-13-α-hydroxy triptolide.

**5.** A triptolide derivative of formula IIIa or IIIb

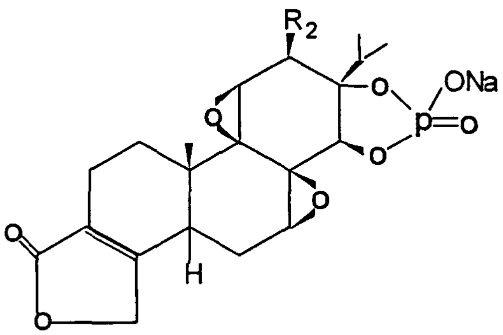

IIIa

or

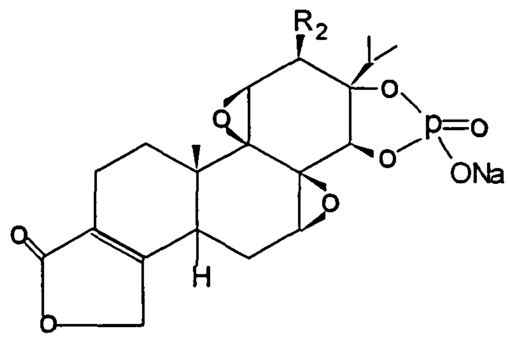

IIIb

Wherein $R_2$ is H, SCN, Cl or Br.

**6.** A method for preparation of the compound according to claim 5, comprising
the step of reacting the second lead compound 12-β-thiocyano-13-α-hydroxy triptolide with $POCl_3$ or other phosphonate halide, phosphate, phosphite and the like, or
the step of reacting the first lead compound triptolide with $POCl_3$ or other phosphonate halide, phosphite halide and the like.

**7.** Use of the compounds according to any one of claims 1, 3, and 5 in the preparation of medicament as immunosuppressive agent or anti-inflammatory agent.

**8.** The use according to claim 7, wherein said immunosuppressive agent or anti-inflammatory agent is used in the treatment of diseases associated with
growth of lymphocytes T and B cells;
production of cytokines such as IL-1, IL-2, IL-6, and iNOS; and
production of Cox-2.

**9.** The use according to claim 8, wherein said diseases are autoimmune deficiency diseases and inflammatory diseases.

**10.** The use according to claim 9, wherein said diseases are selected from the group consisted of rheumatoid arthritis, asthma, systemic lupus erythematosus, psoriasis, multiple sclerosis, atherosclerosis, type I diabetes, and nephritis.

**11.** The use according to claim 7, wherein said immunosuppressive agent or anti-inflammatory agent is used in the prevention of organ rejection and prolongation of the survival time of transplanted organ in organ transplantation.

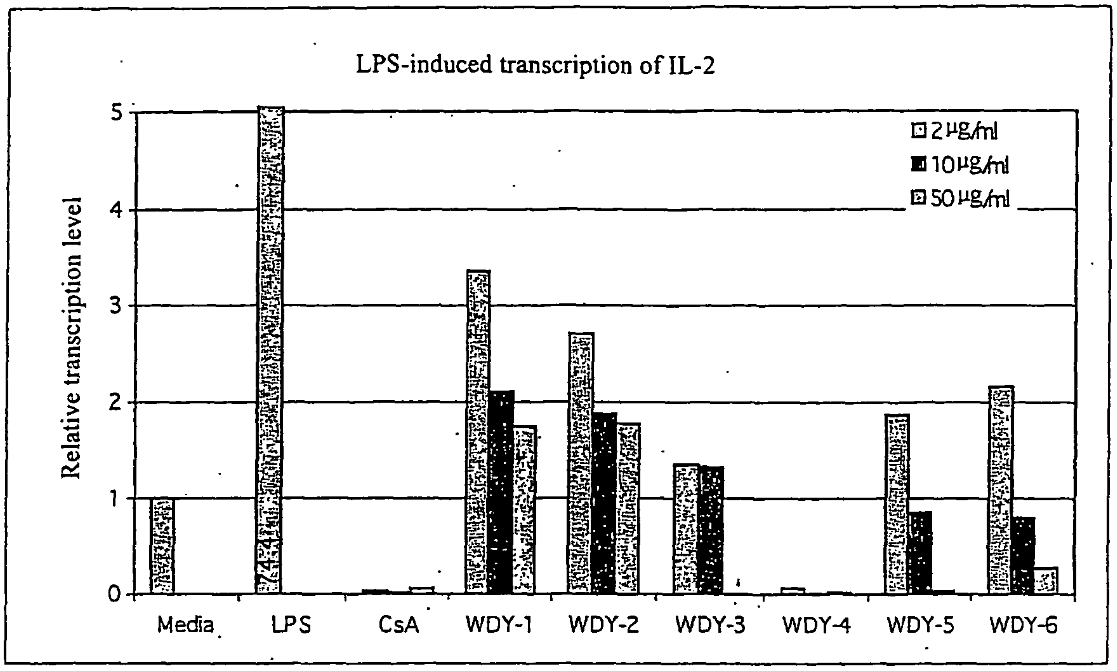
LPS-induced transcription of IL-2
Relative transcription level
0
1
2
3
4
5
2 μg/ml
10 μg/ml
50 μg/ml
Media
LPS
CsA
WDY-1
WDY-2
WDY-3
WDY-4
WDY-5
WDY-6

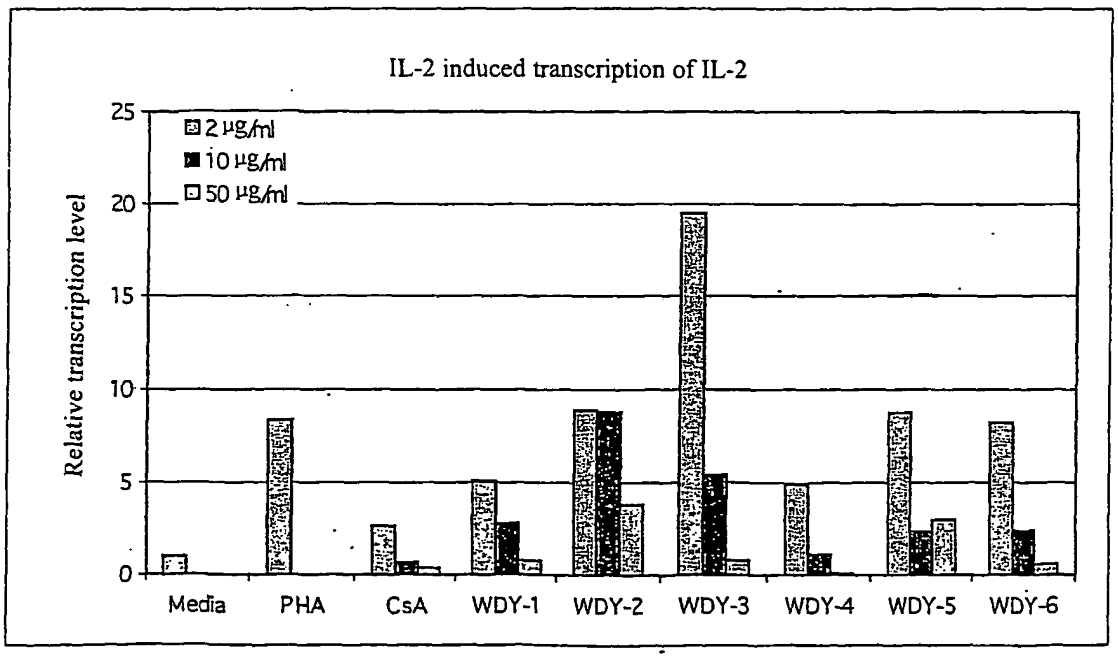
IL-2 induced transcription of IL-2
Relative transcription level
0
5
10
15
20
25
2 μg/ml
10 μg/ml
50 μg/ml
Media
PHA
CsA
WDY-1
WDY-2
WDY-3
WDY-4
WDY-5
WDY-6

Fig.1

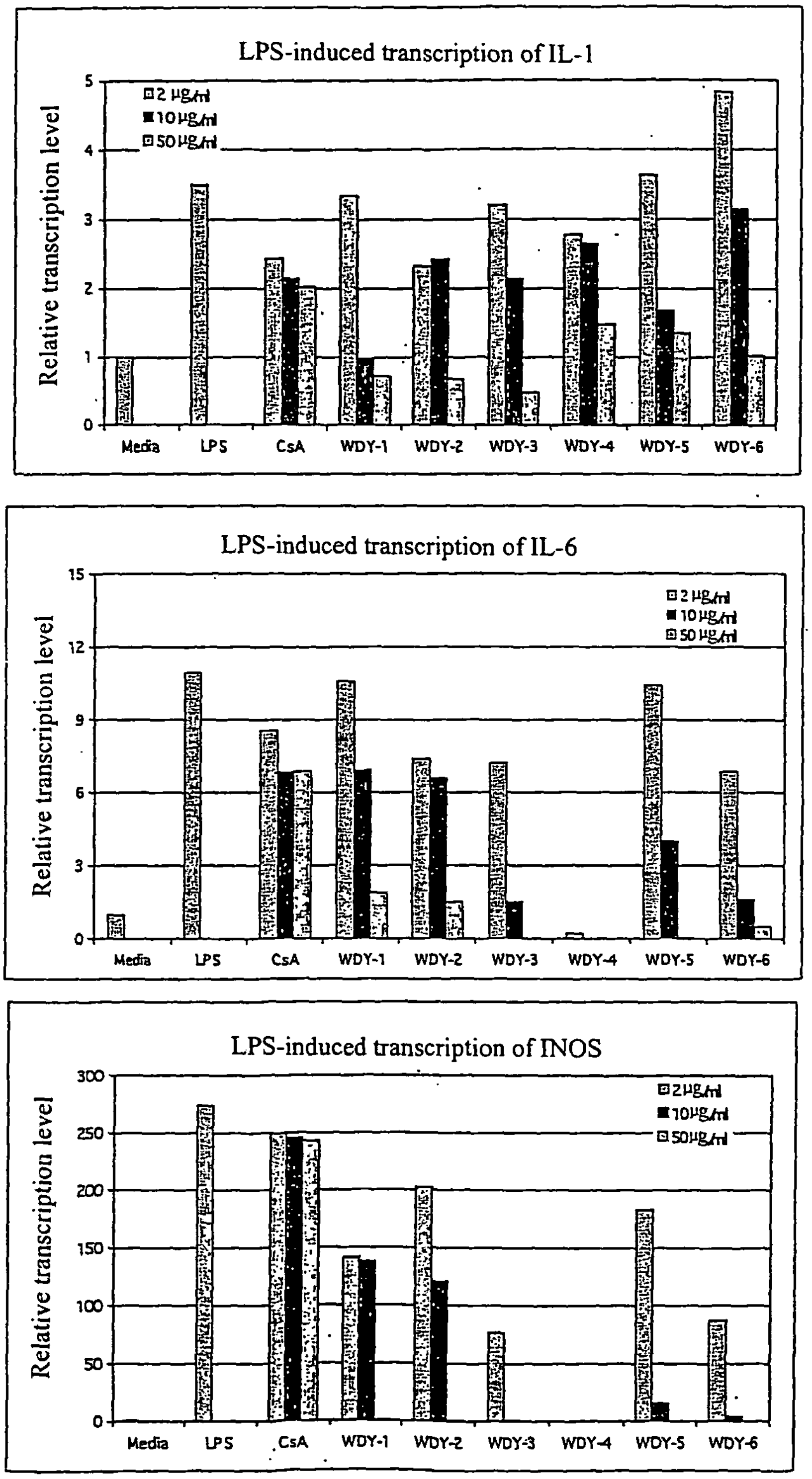
LPS-induced transcription of IL-1
Relative transcription level
2 μg/ml
10 μg/ml
50 μg/ml
Media
LPS
CsA
WDY-1
WDY-2
WDY-3
WDY-4
WDY-5
WDY-6
LPS-induced transcription of IL-6
LPS-induced transcription of INOS

Fig.2

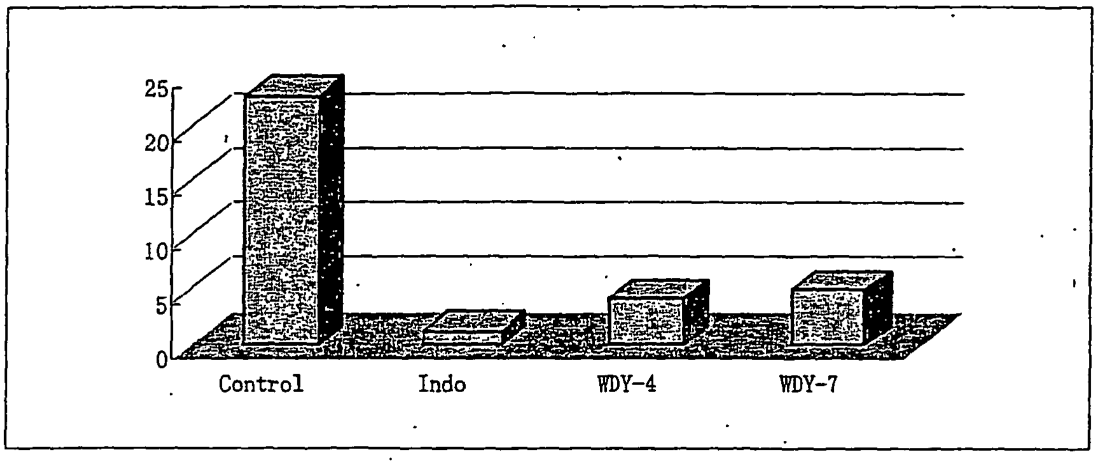


Fig.3

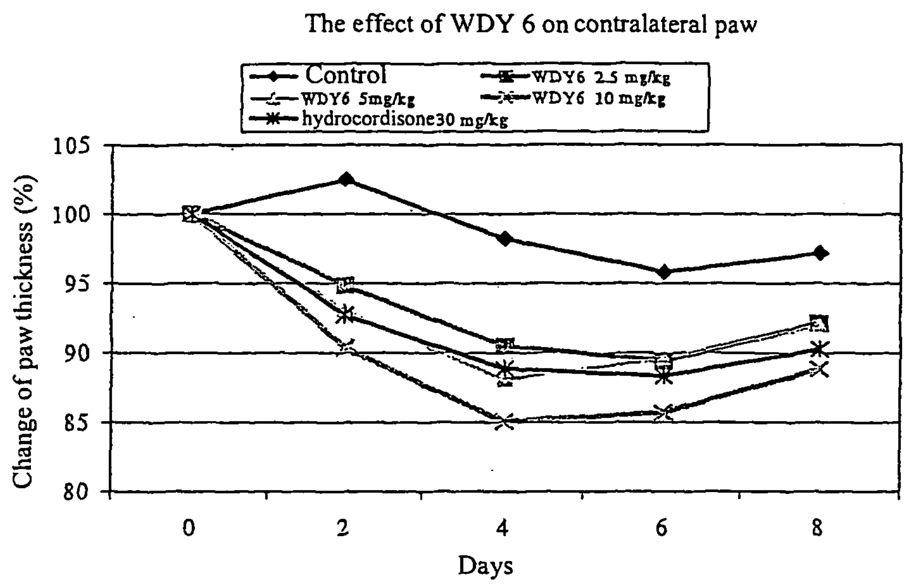
The effect of WDY 6 on contralateral paw
Control
WDY6 2.5 mg/kg
WDY6 5mg/kg
WDY6 10 mg/kg
hydrocordisone30 mg/kg
Change of paw thickness (%)
105
100
95
90
85
80
0
2
4
6
8
Days

Fig.4

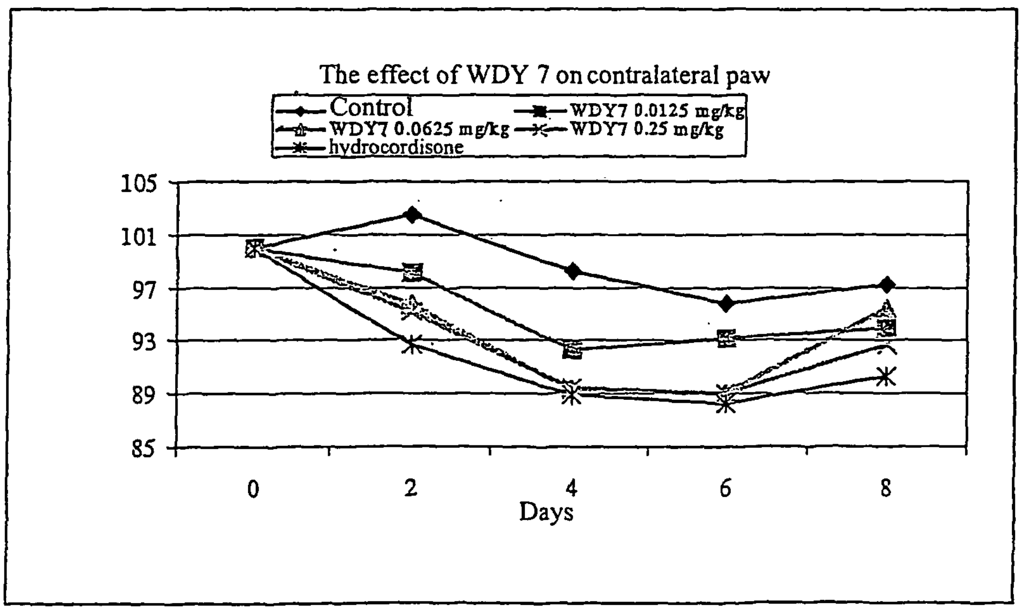
The effect of WDY 7 on contralateral paw
Control
WDY7 0.0125 mg/kg
WDY7 0.0625 mg/kg
WDY7 0.25 mg/kg
hydrocordisone
Change of paw thickness (%)
105
101
97
93
89
85
0
2
4
6
8
Days

Fig.5

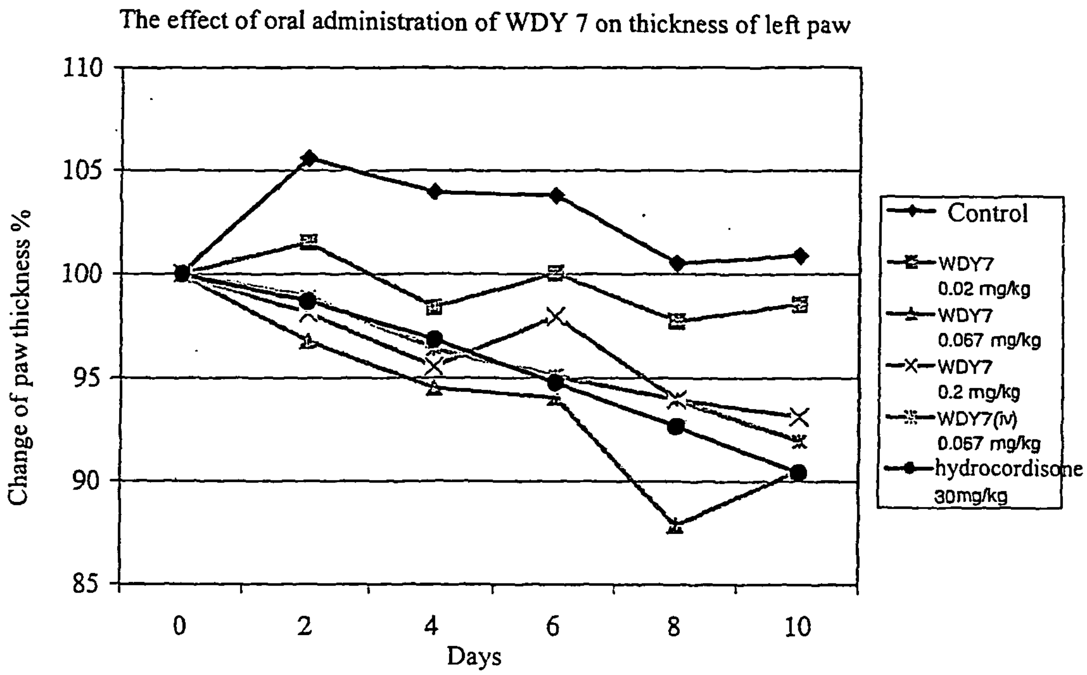
The effect of oral administration of WDY 7 on thickness of left paw
110
105
100
95
90
85
Change of paw thickness %
0
2
4
6
8
10
Days
Control
WDY7 0.02 mg/kg
WDY7 0.067 mg/kg
WDY7 0.2 mg/kg
WDY7(iv) 0.067 mg/kg
hydrocordisone 30mg/kg

Fig.6

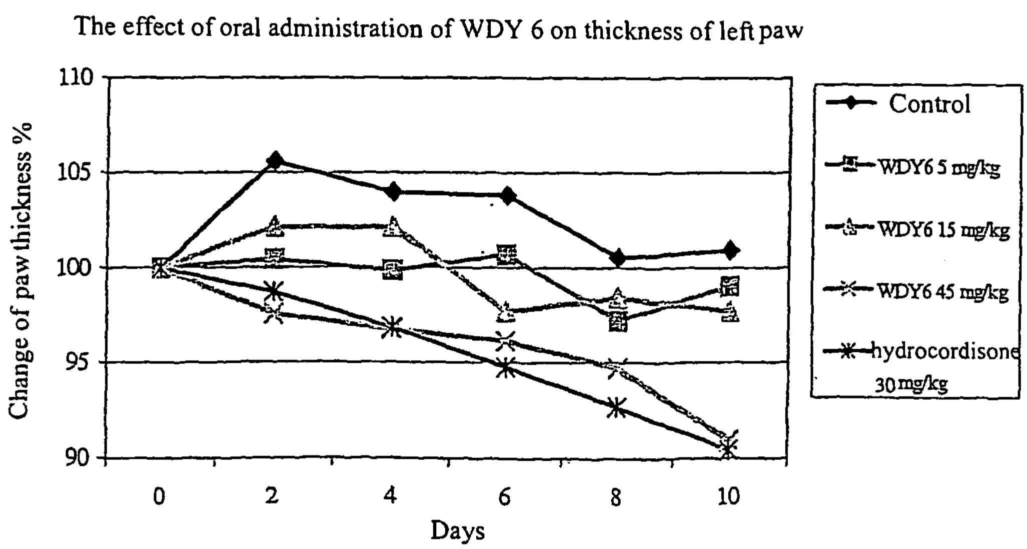
The effect of oral administration of WDY 6 on thickness of left paw
110
105
100
95
90
Change of paw thickness %
0
2
4
6
8
10
Days
Control
WDY6 5 mg/kg
WDY6 15 mg/kg
WDY6 45 mg/kg
hydrocordisone 30 mg/kg

Fig.7

| INTERNATIONAL SEARCH REPORT | International application No. PCT/CN03/00748 |
|---|---|

A. CLASSIFICATION OF SUBJECT MATTER

$IPC^7$: A61K31/335, C07D493/22, A61P37/06

According to International Patent Classification (IPC) or to both national classification and IPC

B. FIELDS SEARCHED

Minimum documentation searched (classification system followed by classification symbols)

A61K, C07D

Documentation searched other than minimum documentation to the extent that such documents are included in the fields searched

Chinese Medical Abstrat, CNKI, CA

Electronic data base consulted during the international search (name of data base and, where practicable, search terms used)

WPI 、 PAJ 、 EPODOC 、 CNKI 、 CNPAT 、 CA

C. DOCUMENTS CONSIDERED TO BE RELEVANT

| Category* | Citation of document, with indication, where appropriate, of the relevant passages | Relevant to claim No. |
|---|---|---|
| X | WO0063212, 26 Sep. 2000, page 1-5, claims | 1-4 |
| A | | 5-11 |

☐ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 23 Sep. 2003(23. 12. 03) | 12 · FEB 2004 (1 2 · 0 2 · 2 0 0 4) |
| Name and mailing address of the ISA/CN<br>6 Xitucheng Rd., Jimen Bridge, Haidian District, 100088 Beijing, China<br>Facsimile No. 86-10-62019451 | Authorized officer<br>Jiang Hui 姜晖<br>Telephone No. 86-10-62085236 |